# EUROPÄISCHE PATENTSCHRIFT

(11) **EP 0 709 367 B1**
(45) Veröffentlichungstag und Bekanntmachung des Hinweises auf die Patenterteilung: **27.08.1997**
(21) Anmeldenummer: 95116459.9
(22) Anmeldetag: 19.10.1995
(51) Int. Cl.: C07C 231/08, C07C 233/03

(54) **Verfahren zur Herstellung von N-Alkenylcarbonsäureamiden**
Process for the preparation of N-alcenyl carboxylic amides
Procédé pour la préparation d'amides carboxyliques N-alkényle

(30) Priorität: 27.10.1994 DE 4438366
(43) Veröffentlichungstag der Anmeldung: 01.05.1996
(73) Patentinhaber: BASF Aktiengesellschaft, 67063 Ludwigshafen (DE)
(72) Erfinder: Heider, Marc, Dr., D-67433 Neustadt (DE); Rühl, Thomas, Dr., D-67227 Frankenthal (DE); Henkelmann, Jochem, Dr., D-68165 Mannheim (DE)

(56) Entgegenhaltungen:
- EP-A- 0 184 074
- FR-A- 2 558 156
- CHEMICAL ABSTRACTS, vol. 120, no. 4, 24.Januar 1994 Columbus, Ohio, US; abstract no. 31326m, SAWAYAMA, S. ET AL 'A new synthetic procedure for N-vinylformamide and free radical polymerization.' Seite 7; Spalte 1;
- PATENT ABSTRACTS OF JAPAN vol. 11 no. 259 (C-441) [2706] ,21.August 1987 & JP-A-62 059248 (MITSUBISHI CHEM IND LTD) 14.März 1987,

## Beschreibung

Die vorliegende Erfindung betrifft ein neues Verfahren zur Herstellung von N-Alkenylcarbonsäureamiden der allgemeinen Formel I 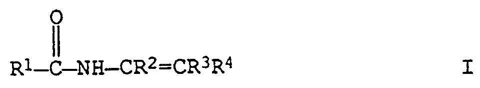 in der die Reste R¹ bis R⁴ unabhängig voneinander für Wasserstoff, aliphatische, cycloaliphatische oder aromatische Reste stehen, die gegebenenfalls inerte Substituenten tragen.

Die Verfahrensprodukte der Formel I sind gesuchte Zwischenprodukte. N-Alkenylcarbonsäureamide können in bekannter Weise polymerisiert werden und anschließend durch Hydrolyse in die entsprechenden Polyvinylamine überführt werden. Diese Polymere, insbesondere Polyvinylformamid, dienen beispielsweise zur Herstellung von Farbstoffen, pharmazeutischen Produkten, Flockungsmitteln und Viskositäts-Stellmitteln in der Papierindustrie (Linhard et al., Das Papier 46/10A (1992), S. V 38-45).

N-Vinylformamid kann durch pyrolytische Spaltung bei ca. 300 bis 400°C aus Ethylidenformamid hergestellt werden. Ethylidenformamid kann beispielsweise unter Quecksilberkatalyse in saurer Lösung aus Formamid und Vinylacetat (DE-A-40 36 097) oder aus Formamid und Acetaldehyd in Gegenwart saurer Katalysatoren hergestellt werden (US-A-4 490 557).

Andere Herstellungswege verlaufen über Zwischenprodukte wie N-Acetylethylformamid (G. Parris, App. Catal., 78 (1991) 65), N-Alkoxyethylformamid (DE-A-3 622 013, DE-A-3 520 829, US-A-4 670 531), N-Hydroxyethylformamid (DE-A-3 500 773) und N-Cyanoethylformamid (DE-A-3 443 463). Diese Verbindungen eliminieren bei relativ hohen Temperaturen von ca. 300 bis 400°C Essigsäure, Alkohole, Wasser bzw. Cyanwasserstoff.

Die genannten Herstellungsverfahren sind zweistufig und erfordern einen thermischen Eliminierungsschritt. Sie sind somit technisch relativ aufwendig und führen aufgrund von Verlusten an Wertprodukt bei den erforderlichen hohen Reaktionstemperaturen nur zu unbefriedigenden Gesamtausbeuten. Diese Herstellmethode ist weiterhin auf verdampfbare Zwischenverbindungen beschränkt, so daß schwerflüchtige Verfahrensprodukte nicht herstellbar sind. Es bestand daher die Aufgabe, ein Verfahren bereitzustellen, das die genannten Nachteile bekannter Verfahren vermeidet.

Demgemäß wurde ein Verfahren zur Herstellung von N-Alkenylcarbonsäureamiden der Formel I gefunden, das dadurch gekennzeichnet ist, daß man ein Amid der allgemeinen Formel II 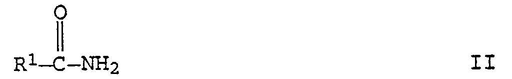 in der der Rest R¹ die oben angegebene Bedeutung hat, und eine Carbonylverbindung der allgemeinen Formel III 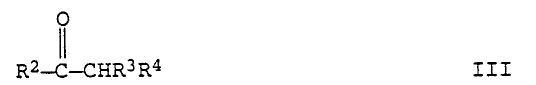 in der die Reste R² bis R⁴ die oben angegebene Bedeutung haben, im Mengenverhältnis von Carbonylverbindung III zum Amid II von 0,5 bis 10 Äquivalenten, in Gegenwart einer Base bei Temperaturen von 0 bis 150°C umsetzt, wobei man
a) die Reaktion entweder in Gegenwart eines Carbonsäurederivats der allgemeinen Formel IV im Mengenverhältnis von Carbonsäurederivat IV zum Amid II von 0,5 bis 10 Äquivalenten vornimmt 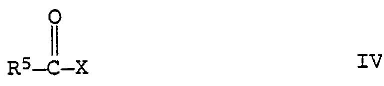 in der der Rest R⁵ für Wasserstoff, eine Alkyl- oder Arylgruppe steht, und X ein Halogen-, Alkoxy- oder Carboxyalkylrest ist,
b) oder in Gegenwart eines Carbonsäurederivats der Formel IV fortsetzt,
und das Amid der Formel I isoliert.

Erfindungsgemäß wird ein Amid der Formel II umgesetzt. Der Rest R¹ kann für Wasserstoff stehen, was bevorzugt ist, weiterhin aber auch für aliphatische Reste, wie Alkylgruppen, beispielsweise C₁-C₆-Alkyl, oder Alkenylgruppen, beispielsweise C₂-C₆-Alkenyl.

Weiterhin kann R¹ für cycloaliphatische Reste wie C₄-C₇-Cycloalkyl oder für C₆-C₁₀-Aryl stehen. Alle Gruppen, insbesondere aber die aromatischen Gruppen, können unter den Reaktionsbedingungen Halogen, Nitro, Alkoxy und Alkyl tragen. Beispielhaft seien als Verbindungen der Formel II Formamid, Acetamid, Propionsäureamid, Crotonsäureamid, Acrylamid und Benzamid genannt.

Ein Amid der Formel II wird mit einer Carbonylverbindung der Formel III umgesetzt. Die Reste R², R³ und R⁴ können für die Gruppen stehen, wie sie für den Rest R¹ oben diskutiert wurden.

Beispielhaft seien Acetaldehyd, Propionaldehyd, Benzaldehyd, Acrolein, Crotonaldehyd, Aceton, Diethylketon, 1-Butanon, Acetophenon und Methylvinylketon genannt, von denen Acetaldehyd und Aceton bevorzugt sind.

Die Menge an Carbonylverbindung im Verhältnis zum eingesetzten Amid kann in weiten Grenzen variieren. In der Regel werden 0,5 bis 10 Äquivalente, bevorzugt 0,8 bis 1,2 Äquivalente Carbonylverbindung pro Äquivalent Amid umgesetzt.

Die Ausgangsverbindungen der Formeln II und III werden in Gegenwart einer Base, bevorzugt einer Brönsted-Base, umgesetzt. Dazu kommen sowohl anorganische wie organische Basen in Betracht. Dabei handelt es sich im einzelnen um Carbonate und Hydrogencarbonate der Alkali- und Erdalkalimetalle wie Natriumcarbonat, Kaliumcarbonat und Natriumhydrogencarbonat, quartäre Ammoniumcarbonate wie Tetramethylammoniumcarbonat, Amide wie Alkalimetallamide, beispielsweise Natriumamid und Kaliumamid, Hydroxide wie Alkalimetallhydroxide, beispielsweise Lithiumhydroxid, Natriumhydroxid und Kaliumhydroxid, Alkalimetallcarboxylate wie Natriumacetat, Alkoholate wie Alkalimetallalkoholate, beispielsweise Natriummethanolat, Natriumethanolat, Kaliummethanolat und Kalium-tert.-butanolat. Kaliumhydroxid kann auch zusammen mit Kronenethern wie 18-Krone-6 verwendet werden.

Als Basen kommen weiterhin Amine wie Ammoniak sowie primäre, sekundäre und tertiäre Amine in Betracht, von denen die tertiären bevorzugt sind. Die Amine können aliphatische, cycloaliphatische oder aromatische Reste tragen, beispielsweise Trialkylamine wie Trioctylamin, Ethyldiisopropylamin, Diethylisopropylamin, Dimethylcyclohexylamin, Triethylamin, außerdem cyclische Amine wie 2,2,6,6-Tetramethylpiperidin, 1,4-Diazabicyclo[2.2.2]octan, 1,8-Diazabicyclo[5.4.0]undec-7-en, 1,5-Diazabicyclo[4.3.0]non-5-en, aliphatische und aromatische Reste tragende Amine wie 1,8-Bis(dimethylamino)-naphthalin und 4-Dimethylaminopyridin und heterocyclische Amine wie N-Alkylimidazole und N-Arylimidazole, z.B. N-Methylimidazol und N-Butylimidazol. Weiterhin kommen Amide wie Dialkylcarbonsäureamide, z.B. Dibutylformamid, in Betracht. Das erfindungsgemäße Verfahren läßt sich auch in Gegenwart von basischen Ionenaustauschern, die in der Regel aus sulfonierten Styrol-Divinylbenzol-Copolymerisaten bestehen wie Amberlite®, Lewatit® und Puralit®, und in Gegenwart von basischen Zeolithen wie Hydrotalcit durchführen.

Weiterhin werden die Ausgangsverbindungen der Formeln II und III mit einem Carbonsäurederivat der Formel IV umgesetzt. Diese tragen einen Rest R⁵, der außer für Wasserstoff für eine Alkylgruppe, vorzugsweise C₁-C₆-Alkyl, oder eine Arylgruppe wie C₆-C₁₀-Aryl, beispielsweise Phenyl, steht. Die Variable X bedeutet Halogen wie Chlor oder Brom, Carboxyalkyl, bevorzugt Carboxy-C₁-C₄-Alkyl, oder bevorzugt einen Alkoxyrest, insbesondere C₁-C₄-Alkoxy wie Ethoxy. Die Verwendung von Estern als Carbonsäurederivat der Formel IV ist gegenüber der Verwendung von Carbonsäurehalogeniden oder Carbonsäureanhydriden bevorzugt, da dabei als Nebenprodukt keine Säuren anfallen, die die eingesetzte Base neutralisieren können.

Es kommen Verbindungen wie Methylformiat, Ethylformiat, Methylacetat, Ethylacetat, Methylpropionat und Ethylpropionat neben Acetanhydrid; Propionsäureanhydrid und Acetylchlorid in Betracht.

Die Menge der eingesetzten Carbonsäurederivate der Formel IV beträgt im allgemeinen 0,5 bis 10 Äquivalente, bevorzugt 0,8 bis 1,2 Äquivalente pro Äquivalent Amid der Formel II.

Die Menge an Base liegt in der Regel bei 0,1 bis 5 Äquivalenten pro Äquivalent Amid der Formel II. Werden Carbonsäureester der Formel IV verwendet, sind 0,5 bis 1,5 Äquivalente Base bevorzugt, im Falle der Carbonsäurehalogenide und -anhydride dagegen 1,5 bis 2,5 Äquivalente Base.

Obwohl die Umsetzung bevorzugt ohne Lösungsmittel ausgeführt wird, kann jedoch ein Lösungsmittel zugesetzt werden, z.B. aprotische Lösungsmittel wie Ether, z.B. Tetrahydrofuran, aromatische Kohlenwasserstoffe wie Toluol und Xylol, weiterhin Acetonitril, Hexamethylphosphorsäuretriamid, Sulfolan und Dimethylsulfoxid. Die Menge liegt im allgemeinen bei 10 bis 90 Gew.-%, bezogen auf den Gesamtansatz.

Die Reaktionstemperatur liegt im allgemeinen bei 0 bis 150°C, bevorzugt bei 20 bis 100°C und besonders bevorzugt bei 30 bis 80°C. In der Regel kann das Verfahren bei Normaldruck ausgeübt werden. Werden jedoch leichtflüchtige Ausgangsverbindungen eingesetzt, hat es sich als zweckmäßig erwiesen, die Umsetzung unter dem jeweiligen Eigendruck der Reaktionsmischung vorzunehmen.

Das erfindungsgemäße Verfahren kann kontinuierlich oder diskontinuierlich ausgeführt werden. Als Reaktoren kommen neben Rührkesseln beispielsweise Rohrreaktoren in Betracht.

Nach der bevorzugten Variante a) wird die Umsetzung als Eintopfreaktion ausgeführt. Die Ausgangsverbindungen der Formeln II, III und IV sowie die Base werden vermischt und auf die Reaktionstemperatur gebracht. Die Reihenfolge des Zusammengebens der Ausgangsverbindungen ist an sich unkritisch. Es hat sich als zweckmäßig erwiesen, Base und Amid der Formel II vorzulegen und mit einem Gemisch aus der Carbonylverbindung der Formel III und des Carbonsäurederivats der Formel IV oder den Einzelkomponenten zu versetzen.

Nach der Verfahrensvariante b) werden zunächst das Amid der Formel II und die Carbonylverbindung der Formel III unter den Reaktionsbedingungen umgesetzt (die Reaktionskontrolle kann gaschromatographisch erfolgen) und anschließend mit dem Carbonsäurederivat der Formel IV weiter umgesetzt.

Die Gesamtreaktion ist in der Regel nach 1 bis 12 h beendet.

Das so erhaltene Reaktionsgemisch kann in an sich bekannter Weise aufgearbeitet werden. Im allgemeinen wird das Produkt destillativ abgetrennt. Der Destillationssumpf kann zur Freisetzung der organischen Basen aus den bei der Reaktion anfallenden Salzen mit starken Basen wie Natronlauge versetzt werden. Die freigesetzten Basen können anschließend extraktiv oder destillativ isoliert werden. Werden in der erfindungsgemäßen Umsetzung leichtflüchtige salzartige Verbindungen wie Formiate tertiärer Ammoniumverbindungen gebildet, können diese auch destillativ aufgearbeitet und in die entsprechenden Amine überführt werden. Die jeweils abgetrennten Basen können in die Reaktion zurückgeführt werden.

Das erfindungsgemäße Verfahren erlaubt die verfahrenstechnisch einfache Herstellung von N-Alkenylcarbonsäureamiden aus leicht zugänglichen Vorprodukten. Die Reaktion läuft bei milden Reaktionstemperaturen ab und führt zu einer hohen Ausbeute an den Verfahrensprodukten. Das Verfahren erlaubt die Herstellung einer Vielzahl verschieden substituierter N-Alkenylcarbonsäureamide.

### Beispiele

### Beispiel 1

Zu einer Mischung aus 22,5 g (0,5 mol) Formamid und 50,5 g Triethylamin wurde bei Raumtemperatur eine Mischung aus 30 g (0,5 mol) Methylformiat und 22 g (0,5 mol) Acetaldehyd innerhalb von 20 Minuten zugetropft. Anschließend erwärmte man 3,5 h auf 40°C. Durch Destillation erhielt man 27 g (76 % d.Th.) Vinylformamid neben 1,3 g Formamid. Überschüssiges Triethylamin wurde zurückgewonnen.

### Beispiel 2

Analog zu Beispiel 1 wurde eine Mischung aus 22,5 g (0,5 mol) Formamid und 50,5 g Triethylamin zunächst bei 10°C mit 22 g (0,5 mol) Acetaldehyd und anschließend bei Raumtemperatur mit 30 g (0,5 mol) Methylformiat umgesetzt. Man erhielt 25,8 g (72,7 % d.Th.) Vinylformamid.

### Beispiel 3

Analog zu Beispiel 1 wurden 22,5 g (0,5 mol) Formamid, 30 g (0,5 mol) Methylformiat und 22 g (0,5 mol) Acetaldehyd in Gegenwart von 53 g (0,5 mol) Na₂CO₃ bei 35°C umgesetzt. Ausbeute: 69 %.

### Beispiel 4

Analog zu Beispiel 1 erhielt man aus 22,5 g (0,5 mol) Formamid, 50,5 g Triethylamin, 30 g (0,5 mol) Methylformiat und 29 g (0,5 mol) Propanal 76 % N-Propenylformamid.

### Beispiel 5

Analog zu Beispiel 1 wurden aus 119 g (1 mol) Benzamid, 110 g (1,09 mol) Triethylamin, 95 g (1,09 mol) Methylformiat und 44 g (1 mol) Acetaldehyd 126 g (87 % d.Th.) N-Vinylbenzamid erhalten.

### Beispiel 6

Analog zu Beispiel 1 wurden 59 g (1 mol) Acetamid, 110 g (1,09 mol) Triethylamin, 44 g Acetaldehyd und 94 g (1,09 mol) Vinylacetat bei 50°C umgesetzt. Ausbeute: 76,6 g (90 % d.Th.).

## Patentansprüche

1. Verfahren zur Herstellung von N-Alkenylcarbonsäureamiden der allgemeinen Formel I 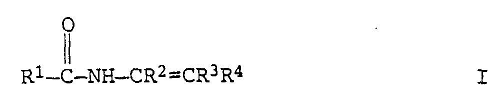 in der die Reste R¹ bis R⁴ unabhängig voneinander für Wasserstoff, aliphatische, cycloaliphatische Reste oder C₆-C₁₀ Aryl stehen, die gegebenenfalls Halogen, Nitro, Alkoxy und Alkyl tragen, dadurch gekennzeichnet, daß man ein Amid der allgemeinen Formel II 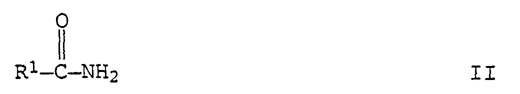 in der der Rest R¹ die oben angegebene Bedeutung hat, und eine Carbonylverbindung der allgemeinen Formel III 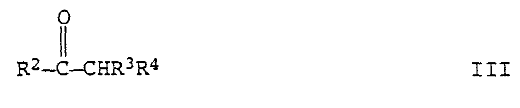 in der die Reste R² bis R⁴ die oben angegebene Bedeutung haben, im Mengenverhältnis von Carbonylverbindung III zum Amid II von 0,5 bis 10 Äquivalenten, in Gegenwart einer Base bei Temperaturen von 0 bis 150°C umsetzt, wobei man
a) die Reaktion entweder in Gegenwart eines Carbonsäurederivats der allgemeinen Formel IV im Mengenverhältnis von Carbonsäurederivat IV zum Amid II von 0,5 bis 10 Äquivalenten vornimmt, 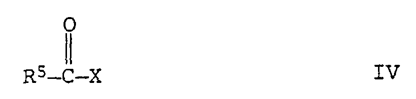 in der der Rest R⁵ für Wasserstoff, eine Alkyl- oder Arylgruppe steht, und X ein Halogen-, Alkoxy- oder Carboxyalkylrest ist,
b) oder in Gegenwart eines Carbonsäurederivats der Formel IV im Mengenverhältnis von Carbonsäurederivat IV zum Amid II von 0,5 bis 10 Äquivalenten fortsetzt,
und das Amid der Formel I isoliert.

2. Verfahren nach Anspruch 1, dadurch gekennzeichnet, daß die Reste R² bis R⁴ für Wasserstoff stehen.

3. Verfahren nach Anspruch 1 oder 2, dadurch gekennzeichnet, daß als Carbonsäurederivat der Formel IV ein Alkylformiat verwendet wird.

4. Verfahren nach den Ansprüchen 1 bis 3, dadurch gekennzeichnet, daß man N-Vinylformamid herstellt.

5. Verfahren nach den Ansprüchen 1 bis 4, dadurch gekennzeichnet, daß man als Base tertiäre Amine einsetzt.

6. Verfahren nach den Ansprüchen 1 bis 5, dadurch gekennzeichnet, daß man die Umsetzung bei 30 bis 80°C vornimmt.

## Claims

1. A process for the preparation of N-alkenylcarboxamides of the formula I 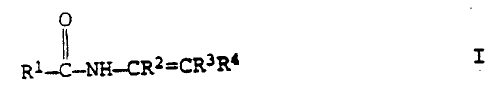 where R¹ to R⁴, independently of one another, are each hydrogen or an aliphatic, cycloaliphatic or C₆-C₁₀-aryl radical which may carry halogen, nitro, alkoxy or alkyl, wherein an amide of the formula II 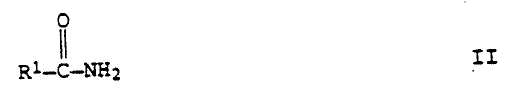 where R¹ has the abovementioned meanings, and a carbonyl compound of the formula III 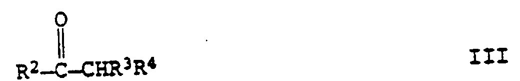 where R² to R⁴ have the abovementioned meanings, are reacted in a ratio of carbonyl compound III to amide II of from 0.5 to 10 equivalents, in the presence of a base at from 0 to 150°C,
a) the reaction either being carried out in the presence of a carboxylic acid derivative of the formula IV 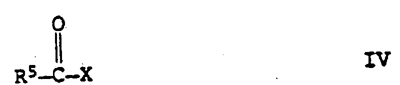 where R⁵ is hydrogen, alkyl or aryl and X is halogen, alkoxy or carboxyalkyl,
in a ratio of carboxylic acid derivative IV to amide II of from 0.5 to 10 equivalents,
b) or being continued in the presence of a carboxylic acid derivative of the formula IV in a ratio of carboxylic acid derivative IV to amide II of from 0.5 to 10 equivalents,
and the amide of the formula I is isolated.

2. A process as claimed in claim 1, wherein R² to R⁴ are each hydrogen.

3. A process as claimed in claim 1 or-2, wherein an alkyl formate is used as carboxylic acid derivative of the formula IV.

4. A process as claimed in any of claims 1 to 3, wherein N-vinylformamide is prepared.

5. A process as claimed in any of claims 1 to 4, wherein the base used is a tertiary amine.

6. A process as claimed in any of claims 1 to 5, wherein the reaction is carried out at from 30 to 80°C.

## Revendications

1. Procédé de préparation d'amides d'acides N-alcénylcarboxyliques de la formule générale I : 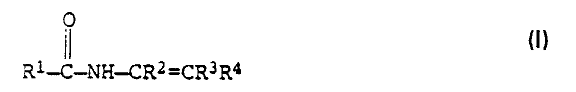 dans laquelle les symboles R¹ à R⁴ représentent, chacun indépendamment l'un de l'autre, un atome d'hydrogène, un radical aliphatique, cycloaliphatique ou aryle en C₆ à C₁₀, qui peut éventuellement porter des atomes d'halogènes, des radicaux nitro, alcoxy et alkyle, caractérisé en ce que l'on fait réagir un amide de la formule générale II : 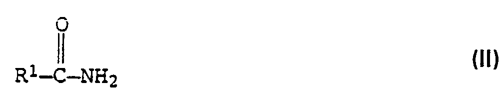 dans laquelle le symbole R¹ possède les significations qui lui ont été attribuées ci-dessus, et un composé carbonylé de la formule générale III : 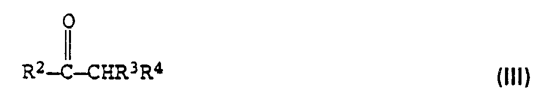 dans laquelle les symboles R² à R⁴ possèdent les significations qui leur ont été attribuées ci-dessus, en un rapport quantitatif du composé carbonylé III à l'amide Il de 0,5 à 10 équivalents, en présence d'une base et à des températures de 0 à 150°C, où on entreprend
a) la réaction en présence d'un dérivé d'acide carboxylique de la formule générale IV dans le rapport quantitatif du dérivé d'acide carboxylique IV à l'amide II de 0,5 à 10 équivalents, 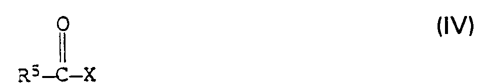 dans laquelle le symbole R⁵ représente un atome d'hydrogène, un radical alkyle ou aryle, et X représente un atome d'halogène, un radical alcoxy ou carboxyalkyle,
b) ou bien on poursuit la réaction en présence d'un dérivé d'acide carboxylique de la formule IV dans le rapport quantitatif du dérivé de l'acide carboxylique IV à l'amide II de 0,5 à 10 équivalents,
et on isole l'amide de la formule I.

2. Procédé suivant la revendication 1, caractérisé en ce que les symboles R² à R⁴ représentent des atomes d'hydrogène.

3. Procédé suivant la revendication 1 ou 2, caractérisé en ce que l'on utilise un formiate d'alkyle à titre de dérivé d'acide carboxylique de la formule IV.

4. Procédé suivant les revendications 1 à 3, caractérisé en ce que l'on prépare le N-vinylformamide.

5. Procédé suivant les revendications 1 à 4, caractérisé en ce que l'on utilise des amines tertiaires à titre de base.

6. Procédé suivant les revendications 1 à 5, caractérisé en ce que l'on entreprend la réaction à une température qui varie de 30 à 80°C.
